(19) Europäisches Patentamt
European Patent Office
Office européen des brevets

(11) Publication number: 0 078 546 B1

(12) EUROPEAN PATENT SPECIFICATION

(45) Date of publication of patent specification: 12.11.86

(21) Application number: 82110158.1

(22) Date of filing: 04.11.82

(51) Int. Cl.[4]: C 07 D 233/90, C 07 D 401/12, A 61 K 31/415, A 61 K 31/44

# (54) Novel imidazolecarboxamide derivatives.

(30) Priority: 04.11.81 US 317958

(43) Date of publication of application: 11.05.83 Bulletin 83/19

(45) Publication of the grant of the patent: 12.11.86 Bulletin 86/46

(84) Designated Contracting States: AT BE CH DE FR IT LI LU NL SE

(56) References cited:
US-A-3 303 199

CHEMICAL ABSTRACTS, vol. 68, no. 19, 6th May 1968, pages 8413,8414, no. 87235f, Columbus, Ohio, USA J. REISCH et al.: "Photochemical transformation of 4-dimethylamino-2,3-dimethyl-1-phenyl-3-pyrazolin-5-one in the presence of formamide"

(73) Proprietor: MERRELL DOW PHARMACEUTICALS INC.
2110 E. Galbraith Road
Cincinnati Ohio 45215 (US)

(72) Inventor: Schnettler, Richard A.
9874 Forest Glen Drive
Cincinnati Ohio 45242 (US)
Inventor: Dage, Richard C.
7825 Shadowhill Way
Cincinnati Ohio 45242 (US)
Inventor: Grisat, Martin J.
9, rue Bain Finkwiller
F-67000 Strasbourg (FR)

(74) Representative: Sgarbi, Renato
GRUPPO LEPETIT S.p.A. Patent & Trademark
Dept. Via Giovanni Durando, 38
I-20158 Milano (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 078 546 B1

**Description**

This invention relates to imidazolecarboxamides and their use as antiallergy agents.

Background Art

2,3-Dihydro-1,5-dimethyl-N-phenyl-2-oxo-1H-imidazole-4-carboxamide is disclosed in Chemical Abstracts, Vol. 68, No. 19, 6th May 1968, pages 8413/8414, No. 87235f.

Certain imidazolone derivatives having at position 1 an aliphatic group such as alkyl, at position 4 a carboxamide group and at position 5 an alkyl group are stated in US—A—3 303 199 to exhibit analgesic, antipyretic, anti-inflammatory and CNS depressant (such as tranquilizing, sedative and muscle relaxant) properties.

Summary of the Invention

This invention is directed to pharmaceutically active imidazolecarboxamides of the general Formula I

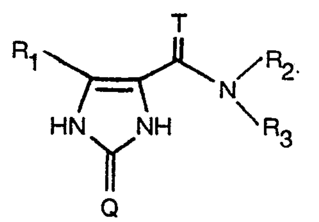

(I)

wherein Q and T are each independently an oxygen atom or a divalent sulfur atom; $R_1$ is hydrogen, $(C_1—C_4)$alkyl, or $CF_3$; $R_2$ is hydrogen, $(C_1—C_4)$alkyl, pyridyl, phenyl, or phenyl substituted at the ortho, meta or para position with $(C_1—C_4)$alkyl, $(C_1—C_4)$alkoxy, hydroxy, halogen, trifluoromethyl, $(C_1—C_4)$alkylthio, $(C_1—C_4)$alkylsulfonyl or $(C_1—C_4)$alkylsulfinyl; $R_3$ is hydrogen or $(C_1—C_4)$alkyl; and the pharmaceutically acceptable salts thereof. These compounds are useful as antiallergy agents.

This invention is also directed to the use of the compounds of formula II

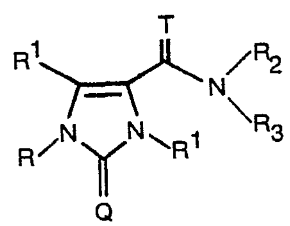

(II)

wherein Q and T are each independently an oxygen atom or a divalent sulfur atom; R and $R^1$ are each independently hydrogen, $(C_1—C_4)$alkyl, $(C_1—C_4)$alkylcarbonyl or benzoyl; $R_1$ is hydrogen, $(C_1—C_4)$alkyl, or $CF_3$; $R_2$ is hydrogen, $(C_1—C_4)$alkyl, pyridyl, phenyl, or phenyl substituted at the ortho, meta or para position with $(C_1—C_4)$alkyl, $(C_1—C_4)$alkoxy, hydroxy, halogen, trifluoromethyl, $C_1—C_4)$alkylthio, $(C_1—C_4)$alkylsulfonyl or $(C_1—C_4)$alkylsulfinyl; $R_3$ is hydrogen or $(C_1—C_4)$alkyl; and the pharmaceutically acceptable salts thereof in the preparation of a medicament for use as an antiallergy agent.

Description of the Preferred Embodiment

As used herein, the term "$(C_1—C_4)$alkyl" means straight and branched chain alkyl of from 1 to 4 carbon atoms such as methyl, ethyl, *n*-propyl, isopropyl, *n*-butyl and isobutyl.

As used herein, the term "$(C_1—C_4)$alkylcarbonyl" is taken to mean a group of the structure

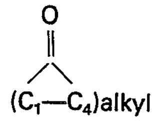

wherein the alkyl moiety is a straight or branched alkyl of from 1 to 4 carbon atoms such as methyl, ethyl, *n*-propyl, isopropyl, *n*-butyl or isobutyl.

As used herein, the term, "benzoyl" is taken to mean a group of the formula $-(CO)C_6H_5$.

As used herein, the term "$(C_1—C_4)$alkoxy" means straight and branched chain alkoxy of from 1 to 4 carbon atoms such as methoxy, ethoxy, *n*-propoxy, isopropoxy, *n*-butoxy or isobutoxy.

As used herein the term "halogen" means fluorine, chlorine, bromine or iodine.

As used herein, the term "halide" means fluoride, chloride, bromide or iodide.

As used herein, the term "pyridyl" means 2-pyridyl, 3-pyridyl and 4-pyridyl.

As used herein, the term "$(C_1—C_4)$alkylthio" means straight and branched chain alkylthio of from 1 to 4 carbon atoms such as methylthio, ethylthio, *n*-propylthio, isopropylthio, *n*-butylthio or isobutylthio.

As used herein, the term "$(C_1—C_4)$alkylsulfonyl" is taken to mean a group of the structure

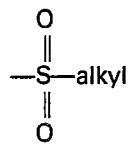

wherein the alkyl moiety is a straight or branched alkyl of from 1 to 4 carbon atoms such as methyl, ethyl, *n*-propyl, isopropyl, *n*-butyl or isobutyl.

As used herein, the term "$(C_1—C_4)$alkylsulfinyl" is taken to mean a group of the structure

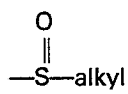

wherein the alkyl moiety is a straight or branched alkyl of from 1 to 4 carbon atoms such as methyl, ethyl, *n*-propyl, isopropyl, *n*-butyl or isobutyl.

The preferred compounds of this invention are those compounds of formula I or II wherein Q and T are oxygen atoms.

The more preferred compounds of this invention are those compounds of formula II wherein R is hydrogen, i.e. the compounds of formula I. Also included among those compounds considered more preferred are those compounds of formula I and II wherein $R_1$ is methyl.

The most preferred compounds of this invention are those compounds of formula I and II wherein $R_2$ and $R_3$ are hydrogen or lower alkyl.

As examples of compounds of formula I and II there may be mentioned the following:

5-methyl-2-oxo-imidazole-4-carboxamide;
5-trifluoromethyl-2-oxo-imidazole-4-carboxamide;
5-ethyl-N-(2-pyridyl)-2-thioxo-imidazole-4-carboxamide;
5-methyl-N-(4-fluorophenyl)-2-oxo-imidazole-4-thiocarboxamide;
5-propyl-N-(3-trifluoromethylphenyl)-2-thioxo-imidazole-4-carboxamide;
5-methyl-N-(3-pyridyl)-2-oxo-imidazole-4-carboxamide;
5-isobutyl-N-(2-methylphenyl)-2-oxo-imidazole-4-carboxamide;
1,3-dimethyl-N-methyl-N-(4-pyridyl)-2-oxo-imidazole-4-carboxamide;
5-(*n*-butyl)-1,3-diacetyl-N-ethyl-N-(3-methoxyphenyl)-2-thioxo-imidazole-4-carboxamide;
1,3-dibenzoyl-N-methyl-N-(4-methylthiophenyl)-2-oxo-imidazole-4-carboxamide;
5-ethyl-N-methyl-N-(2-chlorophenyl)-2-oxo-imidazole-4-carboxamide; and
N,5-dimethyl-2-imidazolinone-4-thiocarboxamide.

Those compounds of formula II wherein R is hydrogen (i.e. the compounds of formula I) are acidic and may form pharmaceutically active salts of formula Ia

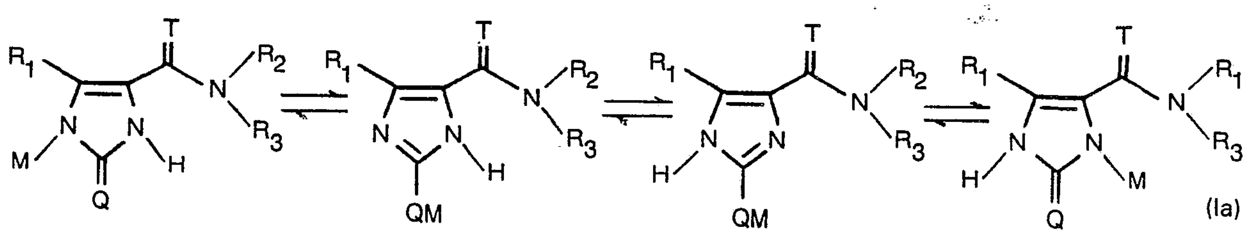

(Ia)

wherein Q, T, $R_1$, and $R_3$ are as defined in formula I, and M is a pharmaceutically acceptable alkali metal such as sodium or potassium; alkaline earth metal such as calcium or magnesium; transition metal such as zinc or iron; or main group metal.

In general, the compounds of this invention are prepared by standard techniques analogously known in the art.

More specifically, the imidazolecarboxamides of this invention wherein T is an oxygen atom and R is hydrogen may be prepared by reaction of an aminoketoamide of formula III

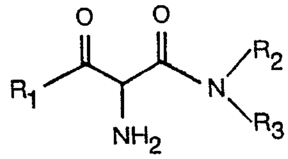

(III)

wherein $R_1$, $R_2$ and $R_3$ are as defined in formula I, with a cyanate or thiocyanate salt as appropriate, preferably a sodium or potassium cyanate or thiocyanate. This reaction is performed by mixing about 1 molar equivalent of the appropriate aminoketoamide with about 1 to about 5 molar equivalents, preferably about 1 molar equivalent, of a cyanate or thiocyanate in a suitable solvent. The reaction is allowed to proceed for about 5 minutes to about 1 hour depending on the reactants, the solvent and the temperature which can be from about −10° to about 50°C, preferably 0°C. Suitable solvents for this reaction are any non-reactive solvents, preferably a water miscible solvent, for example, an organic acid such as acetic acid; an alcohol such as methanol or ethanol; or an ether such as tetrahydrofuran or *p*-dioxan. Preferably the solvent is mixed with water. The preferred solvent is aqueous ethanol.

The product of this reaction may be isolated by any art-known procedure such as by conversion to the corresponding sodium or potassium salt and reprecipitation with carbon dioxide or a mineral acid such as dilute hydrochloric acid.

When it is desired that T be a divalent sulfur atom, the corresponding 2-oxo-imidazolecarboxamide of formula I or II wherein T is an oxygen atom is reacted with phosphorus pentasulfide, $P_2S_5$, by procedures generally known in the art. This reaction may be performed by mixing about 1 molar equivalent of the 2-oxo-imidazolecarboxamide with about 1 to about 5 molar equivalents, preferably about 1 molar equivalent, of $P_2S_5$, together with a suitable solvent. This reaction is allowed to proceed for about 1 to about 10 hours, preferably about 5 hours, depending on the reactant, the solvent and the temperature which can be from 25°C to about 125°C, preferably about 80°C. A suitable solvent for this reaction is any non-reactive solvent, for example, a petroleum ether; a chlorinated hydrocarbon such as chloroform, methylene chloride, carbon tetrachloride or ethylene chloride; an ethereal solvent such as diethyl ether, tetrahydrofuran or *p*-dioxan; or an aromatic solvent such as benzene, toluene or xylene; or pyridine. The preferred solvent is pyridine.

In the compounds of formula I, when desired, one or both of the nitrogen atoms of the imidazolecarboxamide ring may be substituted with an alkyl group by any art-known procedure. Such methods include reacting the appropriate N-unsubstituted imidazolecarboxamide of this invention with a base and an alkylating agent in presence of an unreactive solvent. Suitable bases for this reaction can be, for example, a hydride such as sodium hydride or calcium hydride; a phenoxide such as sodium phenoxide; an alkoxide such as sodium ethoxide; or preferably a hydroxide such as sodium hydroxide. Suitable alkylating agents for this reaction are, for example, an alkyl halide such as methyl iodide; or a dialkylsulfate such as dimethylsulfate. Suitable unreactive solvents are, for example, benzene, toluene, or preferably dimethylformamide (DMF). The reaction is allowed to proceed from about 1 minute to about 1 hour and the temperature may be from about 0°C to about 100°C, preferably about 25°C. When it is desired that only one of the imidazolecarboxamide nitrogen atoms be substituted with an alkyl group, the appropriate imidazolecarboxamide is reacted with from about 1 molar equivalent to about 10 molar equivalents of a base, preferably about 1 molar equivalent and with about 1 molar equivalent of an alkylating agent. Utilizing this procedure, both possible monoalkylated nitrogen isomers result. These isomers are separable by conventional art-known procedures such as fractional crystallization, fractional distillation, or chromatography. When it is desired that both nitrogen atoms of the imidazolecarboxamide ring be alkyl substituted, the appropriate imidazolecarboxamide is reacted with from about 2 molar equivalents to about 10 molar equivalents of a base, preferably about 2 molar equivalents and from about 2 molar equivalents to about 10 molar equivalents of an alkylating agent, preferably about 2 molar equivalents. Finally, any hydroxy substituents, if present, may become alkylated concurrently. That is, when $R_2$ is a phenyl substituted with hydroxy, such group is alkylated under identical reaction conditions. If desired, the alkylation of these substituents may be avoided by the use of suitable protecting groups well-known in the art, for example, hydroxy groups may be benzylated and later deblocked by hydrogenolysis.

Alternatively, in compounds of formula I, when desired, the nitrogen atoms of the imidazolecarboxamide ring may be substituted with an alkylcarbonyl or benzoyl group by any suitable art-known procedure. Such methods include reacting the ring N unsubstituted imidazolecarboxamide of this invention with an acid anhydride. The reactions are allowed to proceed for about 1 to about 20 hours, preferably about 5 hours and the temperature may be from about 0° to about 200°C preferably from 100° to 150°C. Finally, any hydroxy substituents, if present, will become acylated concurrently. That is, when $R_2$ is a phenyl substituted with hydroxy, or when $—NR_2R_3$ is $—NH_2$, such groups are acylated under identical reaction conditions. If desired, the acylation of these substituents may be avoided by the use of suitable protecting groups well-known in the art, for example hydroxy groups may be benzylated and later deblocked by hydrogenolysis.

The alkali metal, alkaline earth metal, transition metal, or main group metal of the imidazolecarboxamides of this invention may be prepared from a corresponding metal salt, for example an alkoxide, such as sodium methoxide or sodium ethoxide; or hydroxides, such as sodium hydroxide or

potassium hydroxide. These reactions may be performed with or without a solvent. Suitable solvents are for example, lower alcohols, such as methanol, ethanol, or *n*-butanol; water or dimethylformamide. The imidazolecarboxamide and base are allowed to react for about 1 minute to about 24 hours depending on the reactants and the temperature which can be from about −78°C to about 150°C, preferably about 0° to about 25°C.

The aminoketoamides of formula III may be prepared by reduction of the appropriate oxime of formula IV

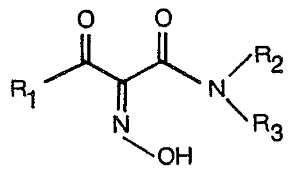

(IV)

wherein $R_1$ and $R_2$ are as defined above in formula I and II. These oximes are reduced by any suitable method generally known in the art such as catalytically in acidic alcoholic medium such as ethanol over an appropriate noble metal catalyst such as palladium or charcoal or with zinc or tin in acetic acid/acetic anhydride solution.

The oximes of formula IV may be prepared by any suitable art-known procedure such as nitrosation of the appropriate amide of formula V

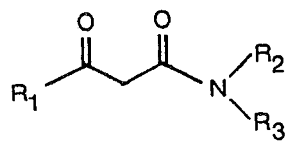

(V)

wherein $R_1$, $R_2$ and $R_3$ are as defined above in formula I and II. Suitable nitrosation reactions are reviewed by O. Tousler in "Organic Reactions", volume VII, pp. 327—377.

The compounds of formula I and II are useful as antiallergy agents and may be administered alone or with suitable pharmaceutical carriers, and can be in solid or liquid form such as, for example, tablets, capsules, powders, solutions, suspensions, or emulsions.

The compounds of this invention can be administered orally, parenterally, for example, subcutaneously, intravenously, intramuscularly, intraperitoneally, by intranasal instillation or by application to mucous membranes such as that of the nose, throat, and bronchial tubes, for example in an aerosol spray containing small particles of a compound of this invention in a spray or dry powder form.

The quantity of novel compound administered will vary. Depending on the patient and the mode of administration, the quantity of novel compound administered may vary over a wide range to provide in a unit dosage of from about 1 to 50 milligrams per kilogram of body weight of the patient per dose to achieve the desired effect. For example, the desired antiallergy effect can be obtained by consumption of a unit dosage form such as, for example, a tablet containing 1 to 50 milligrams of a formula I or II compound of this invention taken 1 to 4 times daily.

The solid unit dosage forms can be of the conventional type. Thus, the solid form can be a capsule which can be the ordinary gelatin type containing a novel compound of this invention and a carrier, for example, lubricant and inert fillers such as lactose, sucrose, corn starch, and the like. In another embodiment, the formula I and II compounds are tabletted with conventional tablet bases such as lactose, sucrose, corn starch, and the like in combination with binders such as acacia, corn starch or gelatin, disintegrating agents such as corn starch, potato starch, or alginic acid, and a lubricant such as stearic acid, or magnesium stearate.

The compounds of formula I and II may also be administered as injectable dosages by solution or suspension of the compounds in a physiologically acceptable diluent with a pharmaceutical carrier which can be a sterile liquid such as water and oils, with or without the addition of a surfactant and other pharmaceutically acceptable adjuvants. Illustrative of oils there can be mentioned those of petroleum, animal, vegetable or synthetic origin, for example, peanut oil, soybean oil, mineral oil, and the like. In general, water, saline, aqueous dextrose, and related sugar solutions, and glycols such as propylene glycol or polyethylene glycol are preferred liquid carriers, particularly for injectable solutions.

For use as aerosols the novel compounds in solution or suspension may be packaged in a pressurized aerosol container together with a gaseous or liquefied propellant, for example, dichlorodifluoromethane, dichlorodifluoromethane with dichlorodifluoroethane, carbon dioxide, nitrogen, propane, etc. with the usual adjuvants such as cosolvents, and wetting agents, as may be necessary or desirable. The compounds may be administered in a nonpressurized form such as in a nebulizer or atomizer.

To illustrate the utility of the formula I and II compounds the following tabulation indicates the reduction of ovalbumin-induced swelling in rat hind paws previously sensitized with rat ovalbumin

antiserum. The test compound (30 mg/kg) is administered orally 15 minutes after i.v. injection of ovalbumin to rats whose hind paws have been sensitized by injection of ovalbumin antiserum 24 hours previously.

| Compound | % Reduction in Swelling |
|---|---|
| 2,3-Dihydro-5-methyl-2-oxo-1H-imidazole-4-carboxamide | 30.8 |
| 2,3-Dihydro-*N,N*,5-trimethyl-2-oxo-1*H*-imidazole-4-carboxamide | 22.6 |
| 2,3-Dihydro-N,N,5-trimethyl-2-thioxo-1*H*-imidazole-4-carboxamide | 22.7 |

The following are illustrative examples of the preparation and use of the compounds of this invention.

Example 1

2,3-Dihydro-N,N,5-trimethyl-2-oxo-1H-imidazole-4-carboxamide

A solution of 15.8 g (0.1 mol) of N,N-dimethyl-2-(hydroxyimino)-3-oxobutanamide in 400 ml of ethanol and 100 ml of 2N hdyrochloric acid over 2 g of catalyst (5% palladium on charcoal) is hydrogenated in a Paar shaker until 1 molar equivalent of hydrogen is taken up (3—5 hours). The catalyst is removed by filtration. A solution of 16.2 g (0.2 mol) of potassium cyanate in 80 ml of water is added, the solution is refluxed for 1 hour and concentrated until the product crystallizes. Recrystallization from 50% aqueous ethanol gives the title compound, m.p. 244—246°C (dec.).

Example 2

2,3-Dihydro-N,5-dimethyl-2-oxo-1H-imidazole-4-carboxamide

Following the procedure of Example 1 but substituting N,N-dimethyl-2-(hydroxyimino)-3-oxo-butanamide by 2-(hydroxyimino)-N-methyl-3-oxo-butanamide gives 2,3-dihydro-N,5-dimethyl-2-oxo-1*H*-imidazole-4-carboxamide, m.p. >300°C.

Example 3

2,3-Dihydro-5-methyl-2-oxo-1H-imidazole-4-carboxamide

Following the procedure of Example 1 but substituting N,N-dimethyl-2-(hdyroxyimino)-3-oxo-butanamide by 2-(hydroxyimino)-3-oxobutanamide gives 2,3-dihydro-5-methyl-2-oxo-1*H*-imidazole-4-carboxamide, m.p. >300°C.

Example 4

N-(tert-Butyl)-2,3-dihydro-5-methyl-2-oxo-1H-imidazole-4-carboxamide

Following the procedure of Example 1 but substituting N,N-dimethyl-2-(hydroxyimino)-3-oxo-butanamide by *N-tert*-butyl-2-(hydroxyimino)-3-oxobutanamide gives 2,3-dihydro-5-methyl-N-(*tert*-butyl)-2-oxo-1*H*-imidazole-4-carboxamide.

Example 5

2,3-Dihydro-5-ethyl-N,N-dimethyl-2-oxo-1H-imidazole-4-carboxamide

Following the procedure of Example 1 but substituting N,N-dimethyl-2-(hydroxyimino)-3-oxo-butanamide by N,N-dimethyl-2-(hydroxyimino)-3-oxo-butanamide gives 2,3-dihydro-5-ethyl-N,N-dimethyl-2-oxo-1H-imidazole-4-carboxamide.

Example 6

2,3-Dihydro-N-(4-methoxyphenyl)-5-methyl-2-oxo-1H-imidazole-4-carboxamide

To a stirred solution of 11.8 g of 2-(hydroxyimino)-N-(4-methoxyphenyl)-3-oxobutanamide in 30 ml of acetic acid and 10 ml of acetic anhydride at 20—30°C is added 10 g of zinc dust and the mixture is stirred for 1 hour. Water (150 ml) is added, the mixture is stirred for 2 hours and the zinc is removed by filtration. The filtrate is evaporated to dryness under reduced pressure and the residue is recrystallized from methanol to give 2-acetyl-amino-N-(4-methoxyphenyl)-3-oxo-butanamide.

This material (10.6 g) is dissolved in 20 ml of 6N hydrochloric acid, the solution is allowed to stand for 5 minutes at room temperature, a solution of 9.7 g of potassium cyanate in 80 ml of water is added and the mixture is stirred at room temperature overnight. The resulting precipitate is recrystallized twice from 50% aqueous ethanol to give the title compound, m.p. 299°—301°C (dec.).

Example 7

2,3-Dihydro-5-methyl-N-phenyl-2-oxo-1H-imidazole-4-carboxamide

Following the procedure of Example 5 but substituting N,N-dimethyl-2-(hydroxyimino)-3-oxo-butanamide by 2-(hydroxyimino)-3-oxo-N-phenylbutanamide gives 2,3-dihydro-5-methyl-N-phenyl-2-oxo-1*H*-imidazole-4-carboxamide.

Example 8

2,3-Dihydro-N-(2,4-dimethylphenyl)-5-methyl-2-oxo-1H-imidazole-4-carboxamide

Following the procedure of Example 5 but substituting N,N-dimethyl-2-(hydroxyimino)-3-oxo-butanamide by N-(2,4-dimethylphneyl)-2-(hydroxyimino)-3-oxo-butanamide gives 2,3-dihydro-N-(2,4-dimethylphenyl)-5-methyl-2-oxo-1*H*-imidazole-4-carboxamide.

Example 9

2,3-Dihydro-N-(4-methoxyphenyl)-5-methyl-2-thioxo-1H-imidazole-4-carboxamide

Following the procedure of Example 6 but substituting potassium cyanate by potassium thiocyanate gives 2,3-dihydro-N-(4-methoxyphenyl)-5-methyl-2-thioxo-1*H*-imidazole-4-carboxamide, m.p. 277—279°C (dec.).

In a like manner substituting 2-(hydroxyimino)-N-methyl-3-oxobutanamide and N,N-dimethyl-2-(hydroxyimino)-3-oxobutanamide for 2-(hydroxyimino)-N-(4-methoxyphenyl)-3-oxobutanamide in the above procedure results in 2,3-dihydro-N,5-dimethyl-2-thioxo-1*H*-imidazole-4-carboxamide, m.p. >300°C, and 2,3-dihydro-*N,N*,5-trimethyl-2-thioxo-1*H*-imidazole-4-carboxamide, m.p. 243—245°C, respectively.

Example 10

2,3-Dihydro-2-oxo-4,N,N-trimethyl-1H-imidazole-5-thiocarboxamide

A suspension of 3.4 g (20 mmol) of 2,3-dihydro-N,N-5-trimethyl-2-oxo-1*H*-imidazole-4-carboxamide (Example 1) and 4.4 g of phosphorus pentasulfide in 100 ml of dry pyridine is stirred at reflux temperature for 6 hours. The mixture is allowed to cool overnight and is diluted with water. The precipitate is collected and recrystallized from 50% aqueous ethanol to give the title compound.

Example 11

2,3-Dihydro-1,3-diisobutyryl-N,N,5-trimethyl-2-oxo-1H-imidazole-4-carboxamide

A mixture of 10.0 g of 2,3-dihydro-N,N,5-trimethyl-2-oxo-1*H*-imidazole-4-carboxamide (Example 1) and 100 ml of isobutyric anhydride are stirred at reflux temperature for 4 hours. During this time a portion of the isobutyric anhydride is distilled off (to remove isobutyric acid that is formed) and is replaced. The mixture is evaporated to dryness under reduced pressure and the residue is crystallized and recrystallized from a mixture of ethyl acetate and hexane to give the title compound.

Example 12

2,3-Dihydro-5-ethyl-N-phenyl-2-oxo-1H-imidazole-4-carboxamide

Following the procedure of Example 5 but substituting N,N-dimethyl-2-(hydroxyimino)-3-oxobutanamide by N,N-dimethyl-2-(hydroxyimino)-3-oxopentanamide gives 2,3-dihydro-5-ethyl-N-phenyl-2-oxo-1*H*-imidazole-4-carboxamide.

Example 13

| Preparation of a Tablet Formulation | Per Tablet |
|---|---|
| (a) 5-Methyl-2-oxo-imidazole-4-carboxamide | 100 mg |
| (b) Cornstarch | 15 mg |
| (c) Lactose | 33.5 mg |
| (d) Magnesium stearate | 1.5 mg |

Example 14

| Preparation of a Parenteral Formulation | Per Tablet |
|---|---|
| (a) N-(4-pyrridyl)-2-oxo-imidazole-4-carboxamide | 1.000 g |
| (b) Polyoxyethylene sorbitan monooleate | 2.000 g |
| (c) Sodium chloride | 0.128 g |
| (d) Water for injection qs ad | 20.000 ml |

**Claims for the Contracting States: BE CH DE FR IT LI LU NL SE**

1. A compound of the formula I

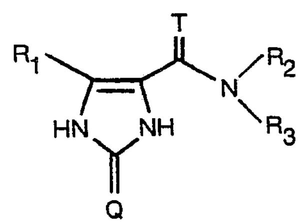

(I)

wherein Q and T are each independently an oxygen atom or a divalent sulfur atom; $R_1$ is hydrogen, $(C_1—C_4)$alkyl, or $CF_3$; $R_2$ is hydrogen, $(C_1—C_4)$alkyl, pyridyl, phenyl, or phenyl substituted at the ortho, meta or para position with $(C_1—C_4)$alkyl, $(C_1—C_4)$alkoxy, hydroxy, halogen, trifluoromethyl, $(C_1—C_4)$alkylthio, $(C_1—C_4)$alkylsulfonyl or $(C_1—C_4)$alkylsulfinyl; $R_3$ is hydrogen or $(C_1—C_4)$alkyl; and the pharmaceutically acceptable salts thereof.

2. A compound of claim 1 wherein Q and T are each an oxygen atom.

3. A compound of claim 2 wherein $R_1$ is methyl.

4. A compound of claim 3 wherein $R_2$ is pyridyl, phenyl or substituted phenyl.

5. A compound of claim 3 wherein $R_2$ and $R_3$ are hydrogen or $(C_1—C_4)$alkyl.

6. A compound of claim 3 wherein $—NR_2R_3$ is $—NH_2$, that is, the compound 2,3-dihydro-5-methyl-2-oxo-1*H*-imidazole-4-carboxamide.

7. A progress for the preparation of an imidazole carboxamide of claim 1 which comprises reacting an aminoketone of the formula III

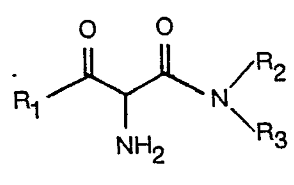

(III)

wherein $R_1$, $R_2$ and $R_3$ are as defined above, with a cyanate or thiocyanate salt; when it is desired that T be a divalent sulfur atom, reacting the thus formed product wherein T is an oxygen atom, with phosphorus pentasulfide; when a pharmaceutically acceptable salt is desired, reacting the thus formed product with a metal basic salt and isolating the imidazolecarboxamide therefrom.

8. A pharmaceutical composition containing at least a compound of claim 1 in admixture with a pharmaceutically acceptable carrier.

9. A compound of claim 1 for use as a medicament.

10. A compound of claim 1 for use as antiallergic agent.

11. Use of a compound of the formula II

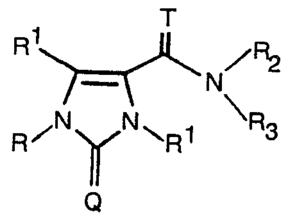

(II)

wherein Q and T are each independently an oxygen atom or a divalent sulfur atom; R and R' are each independently hydrogen, $(C_1—C_4)$alkyl, $(C_1—C_4)$alkylcarbonyl, or benzoyl; $R_1$ is hydrogen, $(C_1—C_4)$alkyl, or $CF_3$; $R_2$ is hydrogen, $(C_1—C_4)$alkyl, pyridyl, phenyl, or phenyl substituted at the ortho, meta or para position with $(C_1—C_4)$alkyl, $(C_1—C_4)$alkoxy, hydroxy, halogen, trifluoromethyl, $(C_1—C_4)$alkylthio, $(C_1—C_4)$alkylsulfonyl or $(C_1—C_4)$alkylsulfinyl; $R_3$ is hydrogen or $(C_1—C_4)$alkyl; and the pharmaceutically acceptable salts thereof for the manufacture of a medicament for antiallergic use.

**Claims for the Contracting States: AT**

1. A process for the preparation of an imidazolecarboxamide of the formula I

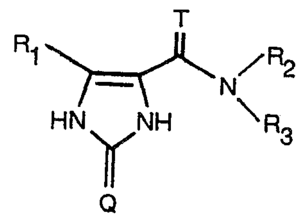

(I)

wherein Q and T are each independently an oxygen atom or a divalent sulfur atom; $R_1$ is hydrogen, $(C_1—C_4)$alkyl, or $CF_3$; $R_2$ is hydrogen, $(C_1—C_4)$alkyl, pyridyl, phenyl, or phenyl substituted at the ortho, meta or para position with $(C_1—C_4)$alkyl, $(C_1—C_4)$alkoxy, hydroxy, halogen, trifluoromethyl, $(C_1—C_4)$alkylthio, $(C_1—C_4)$alkylsulfonyl or $(C_1—C_4)$alkylsulfinyl; $R_3$ is hydrogen or $(C_1—C_4)$alkyl; and the pharmaceutically acceptable salts thereof, which comprises reacting an aminoketone of the formula III

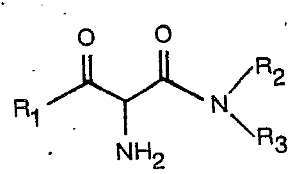

(III)

wherein $R_1$, $R_2$ and $R_3$ are as defined above, with a cyanate or thiocyanate salt; when it is desired that T be a divalent sulfur atom, reacting the thus formed product wherein T is an oxygen atom, with phosphorus pentasulfide; when a pharmaceutically acceptable salt is desired, reacting the thus formed product with a metal basic salt, and isolating the imidazolecarboxamide therefrom.

2. A process according to claim 1 wherein Q and T are each an oxygen atom.

3. A process according to claim 1 wherein Q and T are each an oxygen atom and $R_1$ is methyl.

4. A process according to claim 1 wherein Q and T are each an oxygen atom and $R_2$ is pyridyl, phenyl or substituted phenyl.

5. A process according to claim 1 wherein Q and T are each an oxygen atom and $R_2$ and $R_3$ are hydrogen or $(C_1—C_4)$alkyl.

6. A process according to claim 1 wherein Q and T are each an oxygen atom and $—NR_2R_3$ is $—NH_2$.

7. Use of a compound of the formula II

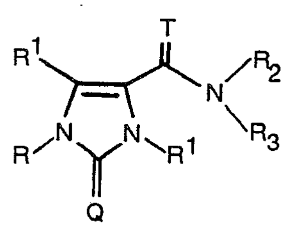

(II)

wherein Q and T are each independently an oxygen atom or a divalent sulfur atom; R and R' are each independently hydrogen, $(C_1—C_4)$alkyl, $(C_1—C_4)$alkylcarbonyl, or benzoyl; $R_1$ is hydrogen, $(C_1—C_4)$alkyl, or $CF_3$; $R_2$ is hydrogen, $(C_1—C_4)$alkyl, pyridyl, phenyl, or phenyl substituted at the ortho, meta or para position

with $(C_1-C_4)$alkyl, $(C_1-C_4)$alkoxy, hydroxy, halogen, trifluoromethyl, $(C_1-C_4)$alkylthio, $(C_1-C_4)$alkylsulfonyl or $(C_1-C_4)$alkylsulfinyl; $R_3$ is hydrogen or $(C_1-C_4)$alkyl; and the parmaceutically acceptable salts thereof for the manufacture of a medicament for antiallergic use.

**Patentansprüche für die Vertragsstaaten: BE CH FR DE IT LI LU NL SE**

1. Verbindung der Formel I

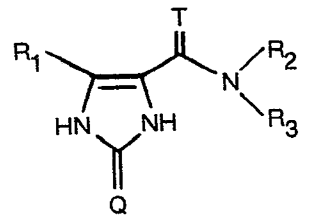

(I)

worin Q and T voneinander unabhängig ein Sauerstoffatom oder ein zweiwertiges Schwefelatom; $R_1$ Wasserstoff, $(C_1-C_4)$-Alkyl oder $CF_3$; $R_2$ Wasserstoff, $(C_1-C_4)$-Alkyl, Pyridyl, Phenyl oder in ortho, meta oder para Position mit $(C_1-C_4)$-Alkyl, $(C_1-C_4)$-Alkoxy, Hydroxy, Halogen, Trifluoromethyl, $(C_1-C_4)$-Alkylthio, $(C_1-C_4)$-Alkylsulfonyl oder $(C_1-C_4)$-Alkylsulfinyl substituiertes Phenyl; und $R_3$ Wasserstoff oder $(C_1-C_4)$-Alkyl bedeuten; und die pharmazeutisch annehmbaren Salze dieser Verbindungen.

2. Verbindung nach Anspruch 1, dadurch gekennzeichnet, daß Q und T beide ein Sauerstoffatom bedeuten.

3. Verbindung nach Anspruch 2, dadurch gekennzeichnet, daß $R_1$ Methyl bedeutet.

4. Verbindung nach Anspruch 3, dadurch gekennzeichnet, daß $R_2$ Pyridyl, Phenyl oder ein substituiertes Phenyl bedeutet.

5. Verbindung nach Anspruch 3, dadurch gekennzeichnet, daß $R_2$ und $R_3$ Wasserstoff oder $(C_1-C_4)$-Alkyl bedeuten.

6. Verbindung nach Anspruch 3, dadurch gekennzeichnet, daß $-NR_2R_3$ $-NH_2$ ist, das heißt, die Verbindung 2,3-Dihydro-5-methyl-2-oxo-1*H*-imidazol-4-carboxamid.

7. Verfahren zur Herstellung eines Imidazolcarboxamides nach Anspruch 1, das die Reaktion eines Aminoketons der Formel III

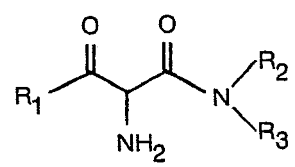

(III)

wobei $R_1$, $R_2$ und $R_3$ wie oben definiert sind, mit einem Cyanat oder Thiocyanatsalz umfaßt; wenn es erwünscht ist, daß T ein zweiwertiges Schwefelatom ist, Reaktion des so gebildeten Produktes, in dem T ein Sauerstoffatom ist, mit Phosphorpentasulfid; wenn ein pharmazeutisch annehmbares Salz erwünscht ist, Reaktion des so gebildeten Produktes mit einem basischen Metallsalz und Isolierung des Imidazolcarboxamid davon.

8. Pharmazeutische Zusammensetzung, die zumindest eine Verbindung nach Anspruch 1 enthält, in Mischung mit einem pharmazeutisch annehmbaren Trägermaterial.

9. Verbindung nach Anspruch 1 zur Verwendung als Medikament.

10. Verbindung nach Anspruch 1 zur Verwendung als antiallergisches Agens.

11. Verwendung einer Verbindung der Formel II

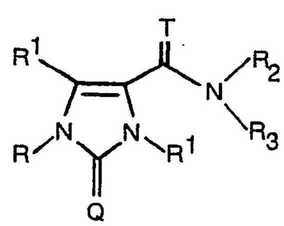

(II)

worin Q und T unabhängig voneinander ein Sauerstoffatom oder ein zweiwertiges Schwefelatom; R und R' unabhängig voneinander Wasserstoff, $(C_1-C_4)$-Alkyl, $(C_1-C_4)$-Alkylcarbonyl oder Benzoyl; $R_1$ Wasserstoff, $(C_1-C_4)$-Alkyl oder $CF_3$; $R_2$ Wasserstoff, $(C_1-C_4)$-Alkyl, Pyridyl, Phenyl oder in ortho, meta oder para Position mit $(C_1-C_4)$-Alkyl, $(C_1-C_4)$-Alkoxy, Hydroxy, Halogen, Trifluoromethyl, $(C_1-C_4)$-Alkylthio,

($C_1$—$C_4$)-Alkylsulfonyl oder ($C_1$—$C_4$)-Alkylsulfinyl; und $R_3$ Wasserstoff oder ($C_1$—$C_4$)-Alkyl bedeuten und die pharmazeutisch annehmbaren Salze dieser Verbindungen zur Herstellung eines Medikamentes zur antiallergischen Verwendung.

**Patentansprüche für den Vertragsstaat: AT**

1. Verfahren zur Herstellung eines Imidazolcarboxamides der Formel I

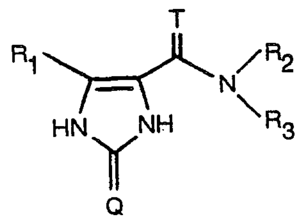

(I)

worin Q and T unabhängig voneinander ein Sauerstoffatom oder ein zweiwertiges Schwefelatom; $R_1$ Wasserstoff, ($C_1$—$C_4$)-Alkyl oder $CF_3$; $R_2$ Wasserstoff, ($C_1$—$C_4$)-Alkyl, Pyridyl, Phenyl oder ein in ortho, meta oder para Position mit ($C_1$—$C_4$)-Alkyl, ($C_1$—$C_4$)-Alkoxy, Hydroxy, Halogen, Trifluoromethyl, ($C_1$—$C_4$)-Alkylthio, ($C_1$—$C_4$)-Alkylsulfonyl oder ($C_1$—$C_4$)-Alkylsulfinyl substituiertes Phenyl; und $R_3$ Wasserstoff oder ($C_1$—$C_4$)-Alkyl bedeuten und der pharmazeutisch annehmbaren Salze dieser Verbindungen umfassend die Reaktion eines Aminoketons der Formel III

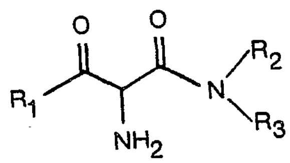

(III)

wobei $R_1$, $R_2$ und $R_3$ wie oben definiert sind, mit einem Cyanat oder Thiocyanat wenn es erwünscht ist, daß T ein zweiwertiges Schwefelatom ist, Reaktion des so gebildeten Produktes, in dem T ein Sauerrstoffatom ist, mit Phosphorpentasulfid: wenn ein pharmazeutisch annehmbares Salz gewünscht wird, Reaktion des so gebildeten Produktes mit einem basischen Metallsalz und Isolierung des Imidazolcarboxamides davon.

2. Verfahren nach Anspruch 1, dadurch gekennzeichnet, daß Q und T beide ein Sauerstoffatom sind.

3. Verfahren nach Anspruch 1, dadurch gekennzeichnet, daß Q und T beide ein Sauerstoffatom sind und $R_1$ Methyl ist.

4. Verfahren nach Anspruch 1, dadurch gekennzeichnet, daß Q und T beide ein Sauerstoffatom sind und $R_2$ Pyridyl, Phenyl oder ein substituiertes Phenyl sind.

5. Verfahren nach Anspruch 1, dadurch gekennzeichnet, daß Q und T beide ein Sauerstoffatom sind und $R_2$ und $R_3$ Wasserstoff oder ($C_1$—$C_4$)-Alkyl sind.

6. Verfahren nach Anspurch 1, dadurch gekennzeichnet, daß Q und T beide ein Sauerstoffatom sind und —$NR_2R_3$, —$NH_2$ ist.

7. Verwendung einer Verbindung der Formel II

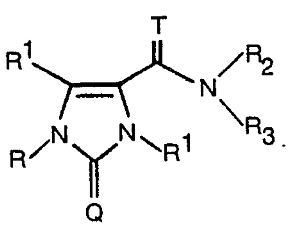

(II)

worin Q und T unabhängig voneinander ein Sauerstoffatom oder ein zweiwertiges Schwefelatom sind; R und R' sind unabhängig voneinander Wasserstoff, ($C_1$—$C_4$)-Alkyl, ($C_1$—$C_4$)-Alkylcarbonyl oder Benzoyl; $R_1$ Wasserstoff, ($C_1$—$C_4$)-Alkyl oder $CF_3$; $R_2$ ist Wasserstoff, ($C_1$—$C_4$)-Alkyl, Pyridyl, Phenyl oder in ortho, meta oder para Position mit ($C_1$—$C_4$)-Alkyl, ($C_1$—$C_4$)-Alkoxy, Hydroxy, Halogen, Trifluoromethyl, ($C_1$—$C_4$)-Alkylthio, ($C_1$—$C_4$)-Alkylsulfonyl oder ($C_1$—$C_4$)-Alkylsulfinyl substituiertes Phenyl; und $R_3$ ist Wasserstoff oder ($C_1$—$C_4$)-Alkyl; und die pharmazeutisch annehmbaren Salze dieser Verbindungen zur Herstellung eines Medikamentes zur antiallergischen Verwendung.

**Revendications pour les Etats contractants: BE CH DE FR IT LI LU NL SE**

1. Composé de formule I:

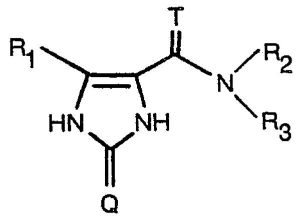

(I)

dans laquelle Q et T représentent chacun, indépendamment, un atome d'oxygène ou un atome de soufre divalent, $R_1$ représente un atome d'hydrogène, un groupe alkyle en $C_1$—$C_4$ ou $CF_3$; $R_2$ représente un atome d'hydrogène, un groupe alkyle en $C_1$—$C_4$, pyridyle, phényle ou phényle substitué en position ortho, méta ou para par un groupe alkyle en $C_1$—$C_4$, alcoxy en $C_1$—$C_4$, hydroxy, halogéno, grifluorométhyle, alkyl (en $C_1$—$C_4$) thio, alkyl (en $C_1$—$C_4$) sulfonyle ou alkyl (en $C_1$—$C_4$) sulfinyle; $R_3$ est un atome d'hydrogène ou un groupe alkyle en $C_1$—$C_4$; et leurs sels pharmaceutiquement acceptables.

2. Composé selon la revendication 1, dans lequel Q et T représentent chacun un atome d'oxygène.

3. Composé selon la revendication 2, dans lequel $R_1$ représente un groupe méthyle.

4. Composé selon la revendication 3, dans lequel $R_2$ représente un groupe pyridyle, phényle ou phényle substitué.

5. Composé selon la revendication 3, dans lequel $R_2$ et $R_3$ représentent chacun un atome d'hydrogène ou un groupe alkyle en $C_1$—$C_4$.

6. Composé selon la revendication 3, dans lequel $—NR_2R_3$ représente $—NH_2$, c'est-à-dire le composé 2,3-dihydro-5-méthyl-2-oxo-1H-imidazole-4-carboxamide.

7. Procédé pour la préparation d'un imidazolecarboxamide de formule I, qui comprend la réaction d'une aminocétone de formule III:

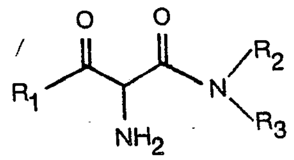

(III)

dans laquelle $R_1$, $R_2$ et $R_3$ sont tels que défini ci-dessus, avec un cyanate ou thiocyanate; quand on désire que R représente un atome de soufre divalent, la réaction du produit ainsi formé, dans lequel T représente un atome d'oxygène, avec le pentansulfure de phosphore; quand on désire un sel pharmaceutiquement acceptable, la réaction du produit ainsi formé avec un sel basique de métal et l'isolement de l'imidazolecarboxamide.

8. Composition pharmaceutique contenant au moins un composé selon la formule I en mélange avec un excipient pharmaceutiquement acceptable.

9. Composé selon la revendication 1, destiné à servir de médicament.

10. Composé selon la revendication 1, destiné à servir d'agent antiallergie.

11. Utilisation d'un composé de formule II:

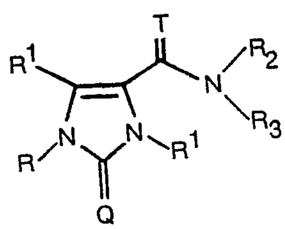

(II)

dans laquelle Q et T représentent chacun, indépendamment, un atome d'oxygène ou un atome de soufre divalent; R et R' représentent chacun, indépendamment, un atome d'hydrogène, un groupe alkyle en $C_1$—$C_4$, alkyl (en $C_1$—$C_4$) carbonyle, ou benzoyle; $R_1$ représente un atome d'hydrogène, un groupe alkyle en $C_1$—$C_4$ ou $CF_3$; $R_2$ représente un atome d'hydrogène, un groupe alkyle en $C_1$—$C_4$, pyridyle, phényle ou phényle substitué en position ortho, méta ou para, par un groupe alkyle en $C_1$—$C_4$; alcoxy en $C_1$—$C_4$, hydroxy, halogéno, trifluorométhyle, alkyl (en $C_1$—$C_4$) thio, alkyl (en $C_1$—$C_4$) sulfonyle ou alkyl (en $C_1$—$C_4$) sulfinyle; $R_3$ représente un atome d'hydrogène ou un groupe alkyle en $C_1$—$C_4$, et leurs sels pharmaceutiquement acceptables, pour la fabrication d'un médicament à utilisation antiallergique.

**Revendications pour L'Etat contractant: AT**

1. Procédé pour la préparation d'un imidazolecarboxamide de formule I:

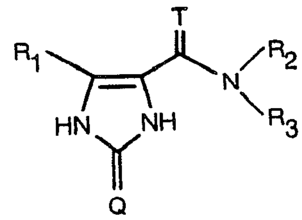

(I)

dans laquelle Q et T représentent chacun, indépendamment, un atome d'oxygène ou un atome de soufre divalent, $R_1$ représente un atome d'hydrogène, un groupe alkyle en $C_1$—$C_4$ ou $CF_3$; $R_2$ représente un atome d'hydrogène, un groupe alkyle en $C_1$—$C_4$, pyridyle, phényle ou phényle substitué en position ortho, méta ou para par un groupe alkyle en $C_1$—$C_4$, alcoxy en $C_1$—$C_4$, hydroxy, halogéno, trifluorométhyle, alkyl (en $C_1$—$C_4$) thio, alkyl (en $C_1$—$C_4$) sulfonyle ou alkyl (en $C_1$—$C_4$) sulfinyle; $R_3$ représente un atome d'hydrogène ou un groupe alkyle en $C_1$—$C_4$; et de leurs sels pharmaceutiquement acceptables, qui comprend la réaction d'une aminocétone de formule III:

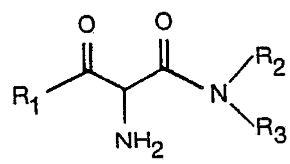

(III)

dans laquelle $R_1$, $R_2$ et $R_3$ sont tels que défini ci-dessus, avec un cyanate ou thiocyanate; quand on désire que T représente un atome de soufre divalent, la réaction du produit ainsi formé, dans lequel T représente un atome d'oxygène, avec le pentasulfure de phosphore; quand on désire un sel pharmaceutiquement acceptable, la réaction du produit ainsi formé avec un sel basique de métal, et l'isolement de l'imidazolecarboxamide.

2. Procédé selon la revendication 1, dans lequel Q et T représentent chacun un atome d'oxygène.

3. Procédé selon la revendication 1, dans lequel Q et T représentent chacun un atome d'oxygène et $R_1$ représente un groupe méthyle.

4. Procédé selon la revendications 1, dans lequel Q et T représentent chacun un atome d'oxygène et $R_2$ représente un groupe pyridyle, phényle ou phényle substitué.

5. Procédé selon la revendication 1, dans lequel Q et T représentent chacun un atome d'hydrogène et $R_2$ et $R_3$ représentent un atome d'hydrogène ou un groupe alkyle en $C_1$—$C_4$.

6. Procédé selon la revendication 1, dans lequel Q et T représentent chacun un atome d'oxygène et —$NR_2R_3$ représente —$NH_2$.

7. Utilisation d'un composé de formule II:

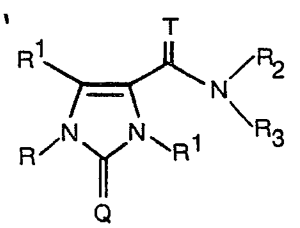

(II)

dans laquelle Q et T représentent chacun, indépendamment, un atome d'oxygène ou un atome de soufre divalent; R et R' représentent chacun, indépendamment, un atome d'hydrogène, un groupe alkyle en $C_1$—$C_4$, alkyl (en $C_1$—$C_4$) carbonyle, ou benzoyle; $R_1$ représente un atome d'hydrogène, un groupe alkyle en $C_1$—$C_4$ ou $CF_3$; $R_2$ représente un atome d'hydrogène, un groupe alkyle en $C_1$—$C_4$, pyridyle, phényle ou phényle substitué en position ortho, méta ou para par un groupe alkyle en $C_1$—$C_4$, alcoxy en $C_1$—$C_4$, hydroxy, halogéno, trifluorométhyle, alkyl (en $C_1$—$C_4$) thio, alkyl (en $C_1$—$C_4$) sulfonyle ou alkyl (en $C_1$—$C_4$) sulfinyle; $R_3$ représente un atome d'hydrogène ou un groupe alkyle en $C_1$—$C_4$, et leurs sels pharmaceutiquement acceptables pour la fabrication d'un médicament à usage antiallergique.